# EUROPÄISCHE PATENTANMELDUNG

(11) **EP 2 926 781 A1**
(43) Veröffentlichungstag der Anmeldung: **07.10.2015**
(21) Anmeldenummer: 14163000.4
(22) Anmeldetag: 01.04.2014
(51) Int. Cl.: A61F 13/00

(54) **Verbandset für die Unterdrucktherapie**

(71) Anmelder: Paul Hartmann AG, 89522 Heidenheim (DE)
(72) Erfinder: Croizat, Pierre, 89542 Herbrechtingen (DE); Eckstein, Axel, 89522 Heidenheim (DE); Hofstetter, Jürgen, 89522 Heidenheim (DE)
(74) Vertreter: Eibl, Christian

(57) **Zusammenfassung**

Verbandset zur Anwendung bei der Unterdrucktherapie von Wunden umfassend eine Wundauflage, ein luftundurchlässiges Abdeckmaterial, ein Unterdruckanschlussmittel sowie eine Sprühdose mit einer filmbildenden Lösung, welche eingesetzt werden kann, um Undichtigkeiten im Unterdruck-Wundverband zu versiegeln.

## Beschreibung

Die vorliegende Erfindung betrifft ein Verbandset zur Anwendung bei der Unterdrucktherapie von Wunden umfassend eine Wundauflage, ein luftundurchlässiges Abdeckmaterial, ein Unterdruckanschlussmittel sowie einer Sprühdose mit einer filmbildenden Lösung, welche eingesetzt werden kann, um Undichtigkeiten im Unterdruck-Wundverband zu versiegeln.

Weiterhin betrifft die Erfindung ein Verfahren zum Abdichten eines auf der Wunde befindlichen Unterdruck-Wundverbandes.

Unterdrucktherapie von Wunden, im Fachbereich auch bezeichnet als topische negative Druckbehandlung (TNP = topical negative pressure) oder NPWT (negative pressure wound therapy), ist seit langem bekannt, findet aber insbesondere seit Mitte/Ende der 1990er Jahre in zunehmendem Maße Anwendung. Dabei wird üblicherweise ein Wundfüllmaterial in die Wunde eingelegt, das Wundgebiet mit einer Folie abgedeckt und mittels eines Drainageschlauchs und einer Vakuumpumpe ein Unterdruck im Wundraum erzeugt. Durch die Verwendung des Wundfüllmaterials wird der Druck gleichmäßig über die Wunde verteilt. Der Unterdruck bewirkt eine effektive Wundreinigung durch Abtransport von Wundexsudat. Als weitere Vorteile werden Förderung der Bildung von Granulationsgewebe und Verminderung der Wundödembildung genannt. Wie der Name VAC bereits suggeriert, wird die Unterdrucktherapie üblicherweise bis zum Wundverschluß durchgeführt. Der Wundverschluß gilt im Allgemeinen als erreicht, wenn über der vormaligen Wundstelle eine Schicht Epidermis vorhanden ist. Ein Wundverschluß kann durch Heilungsprozesse oder durch chirurgische Eingriffe erreicht werden.

Unterdrucktherapie wird insbesondere eingesetzt bei akuten oder chronischen Wunden, beispielsweise bei Geschwüren (z.B. Druckgeschwüren, diabetischen, neuropathischen oder venös bedingten Geschwüren), traumatischen Wunden, therapieresistenten Wunden, infizierten Wunden, postoperativen Wunden, sondierten Fisteln sowie Hautlappen und Hauttransplantaten. Chronische Wunden sind durch eine verlangsamte oder fehlende Wundheilung gekennzeichnet.

Vorrichtungen zur Unterdrucktherapie von Wunden sind entsprechend im Stand der Technik bekannt. So beschreibt beispielsweise die WO 1993/009727 eine Vorrichtung zur Förderung der Wundheilung durch die Applikation von Unterdruck auf dem die Wunde aufweisenden und die Wunde umgebenden Hautbereich. Die Vorrichtung gemäß WO 1993/0097271 umfasst eine Vakuumeinrichtung zur Erzeugung des Unterdrucks, eine luftdichte Abdeckung der Wunde, welche mit der Vakuumeinrichtung in einer funktionellen Verbindung steht, sowie eine Wundauflage zur Positionierung an der Wunde innerhalb der luftdichten Abdeckung. Bei der Wundauflage handelt es sich vorzugsweise um einen offenzelligen Polymer-Schaumstoff, beispielsweise um Polyester-Schaum. Neben der Verwendung von offenzelligem Polymer-Schaumstoff wurden andere Materialien zur Herstellung von Wundauflagen für die Unterdrucktherapie von Wunden beschrieben.

Geräte zur Unterdrucktherapie von Wunden sind auch kommerziell erhältlich und umfassen kleine, tragbare Geräte, die den Patienten eine gewisse Mobilität ermöglichen bis hin zu stationär zu verwendenden Geräten, etwa in Langzeitpflegeeinrichtungen.

Die Unterdrucktherapie kann zur Behandlung einer Vielzahl unterschiedlicher Wundtypen eingesetzt werden, beispielsweise Druckwunden (Dekubitus), Geschwüren (Ulcus), Brandwunden oder Wunden infolge traumatischer Einwirkung. Insbesondere findet die Unterdrucktherapie Anwendung bei der Behandlung schlecht heilender und/oder infizierter Wunden.

Ein bei der praktischen Anwendung der Unterdrucktherapie häufig auftretendes Problem stellt die luftdichte Abdichtung des Verbandes dar. Probleme beim Abdichten des Unterdruck-Verbandes können sich sowohl beim Anliegen des Verbandes, als auch während der Unterdrucktherapie ergeben. Während der Unterdrucktherapie auftretende Undichtigkeiten können beispielsweise durch mechanische Beanspruchung des Verbandes oder durch Schweißabsonderungen entstehen. Aus dem Stand der Technik sind Hilfsmittel zur luftdichten Versiegelung des Wundbereiches beim Anliegen des Verbandes bekannt. So schlägt beispielsweise die WO1996009022 vor, beim Anlegen des Unterdruck-Verbandes eine adhäsive Dichtmasse zwischen Haut und Abdeckfolie einzubringen. Die WO2009156984 beschreibt ein fließfähiges Dichtmaterial, welches beispielsweise aus einer Tube appliziert werden kann. Die DE102010012521 schlägt vor, den als Wundauflage verwendeten Saugkörper mit einer polymerisierbaren Klebstoffzusammensetzung zu überziehen, um hierdurch eine Abdichtung des Wundraumes zu erreichen.

Aus dem Stand der Technik sind gleichfalls Mittel bekannt, um während der Wundtherapie auftretende Undichtigkeiten am Unterdruck-Verband zu erkennen und gegebenenfalls abzudichten. Die WO2011123624 offenbart ein auf einer elektronischen Kamera basierendes Imaging-System, um undichte Stellen an der Peripherie des Verbandes oder in der Abdeckfolie zu identifizieren. Bei dem in der WO2013039622 vorgestellten System sollen Undichtigkeiten anhand eines Detektions-Materials, welches bei Luftkontakt seine Farbe ändert, erkennbar werden. Aus der WO2013066775 ist ein Richtmikrofon bekannt, mittels dem Lecks im Verband anhand des während der Therapie durch die Undichtigkeit verursachten Geräusches lokalisiert werden können. Zur Versiegelung von während der Unterdruck-Therapie auftretenden Undichtigkeiten schlägt die WO2003057307 Reparatur-Pflaster vor, die in unterschiedlichen Größen bereitgestellt werden.

Der vorliegenden Erfindung liegt die Aufgabe zugrunde, ein Mittel bereitzustellen, welches geeignet ist, während der Unterdruck-Therapie auftretende Undichtigkeiten schnell und einfach zu versiegeln. Insbesondere soll das Abdichtungs-Mittel zum unkomplizierten Verschließen kleiner Leckagestellen im Verband geeignet sein. Zudem war es eine Aufgabe, die Erkennung von Undichtigkeiten im Verband zu vereinfachen. Eine Abdichtung des Wundverbandes sollte jedoch idealerweise auch dann möglich sein, wenn die genaue Lokalisation der Leckage nicht bekannt ist.

Es wurde nun gefunden, dass eine sehr effektive Versiegelung von kleineren Leckagestellen im Unterdruck-Verband mittels einer aus einer Sprühdose (auch als Spraydose bezeichnet) auf dem Verband gesprühten filmbildenden Lösung erreicht werden kann. Hierzu ist lediglich erforderlich, dass die Unterdruckquelle vor dem Auftrag der filmbildenden Lösung deaktiviert wird, um ein Absaugen der Lösung durch die Leckagestelle in den Wundraum zu unterbinden, bevor sich der die Undichtigkeit versiegelnde Film ausgebildet hat. Des weiteren wurde gefunden, dass die dichtende Wirkung der aufgesprühten Lösung noch weiter verbessert werden kann, wenn die Lösung nach dem Auftrag auf den Verband verrieben wird, beispielsweise mit einem Finger oder mit einem nicht saugfähigen Tuch.

Erfindungsgemäß vorgeschlagen wird somit die Verwendung einer Sprühdose beinhaltend eine filmbildende Lösung zum Abdichten eines auf der Wunde befindlichen Unterdruck-Wundverbandes.

Es hat sich als praktisch erwiesen, die Sprühdose mit der filmbildenden Lösung in Verbindung mit einem Verbandset bereitzustellen, welches sämtliche für die Unterdrucktherapie benötigten Verbandkomponenten umfasst. Gegenstand der Erfindung ist deshalb auch ein Verbandset, geeignet zur Anwendung bei der Unterdrucktherapie von Wunden, umfassend eine Wundauflage zum Einbringen in den Wundraum, ein luftundurchlässiges Abdeckmaterial zum luftdichten Verschließen der Wunde und der Wundumgebung, ein Unterdruckanschlussmittel (üblicherweise als "Port" oder "Unterdruckport" bezeichnet) zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind, sowie eine Sprühdose beinhaltend eine filmbildende Lösung zum Abdichten eines auf der Wunde befindlichen Unterdruck-Wundverbandes, wobei die filmbildende Lösung auf das Abdeckmaterial aufgesprüht werden kann, um Undichtigkeiten im Verband zu versiegeln.

Gegenstand der Erfindung ist gleichfalls ein Verfahren zum Abdichten eines auf der Wunde befindlichen Unterdruck-Wundverbandes, umfassend die Schritte i) Erkennen einer undichten Stelle am Unterdruck-Wundverband, ii) Deaktivieren der Unterdruckquelle und Belüften des Verbandes, so dass im Wundraum weitestgehend Umgebungsdruck vorhanden ist, iii) Aufsprühen einer filmbildenden Lösung auf die undichte Stelle des Unterdruck-Wundverbandes, wobei das Aufsprühen somit erfolgt, während kein Unterdruck an der Wunde angelegt ist, iv) gegebenenfalls Verreiben der filmbildenden Lösung auf dem Unterdruck-Wundverband sowie v) Abwarten für mindestens 30 Sekunden, bis sich ein die Leckagestelle abdichtender Film ausgebildet hat und schließlich vi) Fortsetzen der Unterdrucktherapie.

Das vorgeschlagene Verfahren ist unkompliziert und schnell anzuwenden. Es eignet sich insbesondere zur Abdichtung kleinerer Beschädigungen am Abdeckmaterial, beispielsweise kleinere Löcher, die während der Unterdruck-Behandlung durch das Einwirken spitzer Gegenstände oder durch mechanische Beanspruchung entstanden sind. Ein besonderer Vorteil des Verfahrens besteht darin, dass der Unterdruck-Verband auch dann wirksam abgedichtet werden kann, wenn die undichte Stelle nicht lokalisiert werden konnte. In diesem Falle kann der gesamte Verband mit der filmbildenden Lösung besprüht werden.

Das mit der vorliegenden Erfindung vorgeschlagene Verfahren eignet sich besonders zur Abdichtung von Leckagen an einem Unterdruck-Wundverband, die während der Therapie auftreten. Grundsätzlich kann die filmbildende Lösung jedoch unmittelbar nach dem Anlegen des Verbandes zum Einsatz kommen. Hierzu kann die Oberfläche der Abdeckfolie besprüht werden, um Leckagen in der Folie abzudichten. Gleichfalls kann der Randbereich der Abdeckfolie, welcher an die die Wunde umgebende Haut angrenzt, mit der filmbildenden Lösung besprüht werden, um Undichtigkeiten verursachende Kanäle zwischen Folie und der Haut des Patienten zu verschließen.

Bei an einem Wundsimulator (ein geeigneter Wundsimulator ist beispielsweise in der WO2010/072349 beschrieben) durchgeführten Untersuchungen wurde herausgefunden, dass Öffnungen in der Abdeckfolie, welche einen Durchmesser von beispielsweise 1 bis 2 mm aufweisen, unter Verwendung des erfindungsgemäß vorgeschlagenen Verfahrens zuverlässig abgedichtet werden können, wenn eine geeignete filmbildende Lösung aufgesprüht wird. Die abdichtende Wirkung kann weiter verbessert werden, wenn der SprühAuftrag wiederholt durchgeführt wird (beispielsweise zwei bis drei Mal) und/oder wenn die filmbildende Lösung nach dem Auftrag auf der Abdeckfolie unter geringem Andruck kurz an der Auftragsstelle verrieben wird. Zweckmäßigerweise kann das Verreiben mit einem Finger oder mit einem nicht saugfähigen Tuch erfolgen.

Die filmbildende Lösung umfasst insbesondere eine polymerisierbare Substanz, welche nach dem Aufsprühen der Lösung auf den Verband einen das Abdeckmaterial versiegelnden Polymerfilm bildet. Bei der polymerisierbaren Substanz kann es sich um eine synthetische Substanz oder um eine natürliche Substanz handeln.

Im Zusammenhang mit der Erfindung ist es erforderlich, dass die aufgesprühte Lösung in weniger als 3 Minuten, bevorzugt in weniger als 1 Minute, besonders bevorzugt in weniger als 30 Sekunden, einen dichtenden Film auf der Verbandoberfläche ausbildet. Die Filmbildung erfolgt üblicherweise durch die Polymerisation einer in der aufgesprühten Lösung enthaltenen polymerisierbaren Substanz nach Luftkontakt.

Der durch die polymerisierbare Substanz auf der Verbandoberfläche gebildete Polymerfilm kann beispielsweise Naturkautschuk, Nitrocellulose, Polyurethan, Polyurethan-Copolymer, Polyurethan-Polyharnstoff-Copolymer, Polyacrylat, Polymethacrylat oder ein Copolymer auf Acrylat oder Methacrylatbasis umfassen.

Im Zusammenhang mit der Erfindung besonders geeignete polymerisierbare synthetische Substanzen umfassen Acrylsäure, Methacrylsäure, Vinylchlorid oder ein Derivat dieser Stoffe.

Grundsätzlich wäre denkbar, dass die Filmbildung durch einen Polymerisationsinitiator, insbesondere einen Katalysator oder einen Polymerisationsbeschleuniger, aktiviert wird. Hierzu ist es normalerweise erforderlich, den Polymerisationsinitiator getrennt von der polymerisierbaren Substanz bereitzustellen. Dies kann beispielsweise durch eine Doppelkammer-Sprühdose erreicht werden, wobei eine Kammer die polymerisierbare Substanz und eine weitere Kammer den Polymerisationsinitiator enthält.

In der Spraydose liegt die polymerisierbare Substanz üblicherweise in einem Lösungsmittel, beispielsweise in Wasser, Ethanol, Aceton, Ethylacetat, Dimethylether oder einem Gemisch daraus vor.

Des weiteren kann die filmbildende Lösung einen Weichmacher, ein Netzmittel und/oder einen Emulgator umfassen. Gegebenenfalls kann auch eine antimikrobiell wirksame Substanz enthalten sein, beispielsweise Triclosan.

Als Treibgase können beispielsweise Propan, Butan, Dimethylether oder Gemische daraus eingesetzt werden. Gegebenenfalls kann auch komprimierte Luft oder Stickstoff zum Einsatz kommen.

Es hat sich herausgestellt, dass im Zusammenhang mit der Erfindung auch so genannte Sprühpflaster eingesetzt werden können. Derartige Sprühpflaster sind beispielsweise unter der Bezeichnung "Hansaplast Sprüh-Pflaster" (Beiersdorf AG, Deutschland), "flint MED Sprühverband" (Togal Werk AG, Deutschland), "newaid Sprühpflaster (New Pharma AG, Schweiz) oder "URGO Sprühpflaster" (Urgo GmbH, Deutschland) im Handel. In Vergleichsversuchen hat sich herausgestellt, dass der "flint MED Sprühverband" besonders vorteilhaft zur Abdichtung von kleineren Öffnungen (Durchmesser ca. 1 bis 2 mm) in einer aus Polyurethan bestehenden Abdeckfolie eingesetzt werden kann. Der "flint MED Sprühverband" enthält Poly (butylmethacrylat, methylmethacrylat) als polymerisierbare Substanz, sowie Butan, Ethylacetat, Propan und natives Rizinusöl als Hilfsstoffe.

Im Zusammenhang mit der Erfindung gleichfalls einsetzbare versprühbare Mischpolymerisatlösungen auf der Basis von Isobuten, Acrylsäure oder Methacrylsäureestern sowie Maleinsäuremonoalkyester sind in der DE2343923 beschrieben.

Gemäß einer bevorzugten Ausführungsform wird ein Verbandset vorgeschlagen, wobei das Verbandset weiterhin ein Mittel zur Detektion einer undichten Stelle am Verband umfasst. Bei dem Mittel zur Detektion einer undichten Stelle am Verband kann es sich im einfachsten Falle um aufgesprühtes steriles Wasser, welches vorzugsweise ein Netzmittel umfasst, handeln. Das Aufsprühen muss hierbei während des laufenden Unterdruckbetriebes erfolgen, d.h. während Unterdruck am Verband angelegt ist. Nach dem Aufsprühen des Wassers kann die undichte Stelle mit bloßem Auge erkannt werden, da im Bereich der Undichtigkeit das Wasser in den Verband hinein abgesaugt wird. Die Erkennbarkeit der Undichtigkeit kann weiter verbessert werden, wenn dem sterilen Wasser ein mit bloßem Auge erkennbarer Farbstoff zugesetzt wird. Das Eindringen der gefärbten Flüssigkeit in den Verband kann besonders gut mit bloßem Auge erkannt werden, wenn eine weiße oder helle Wundauflage, beispielsweise ein weißer Schaum, eingesetzt wird. Alternativ kann das sterile Wasser oder die sterile wässrige Lösung einen medizinisch verträglichen Fluoreszenz-Farbstoff, beispielsweise Chinin, enthalten. Die Leckagestelle kann dann unter UV Beleuchtung erkannt werden.

Das sterile Wasser, welches optional einen mit bloßem Auge erkennbaren Farbstoff oder einen Fluoreszenz-Farbstoff umfasst, kann beispielsweise in Form einer Spraydose oder eines Pumpsprays bereitgestellt werden.

Weitere geeignete Mittel zur Detektion einer undichten Stelle am Verband sind beispielsweise in den eingangs erwähnten Dokumenten WO2011123624, WO2013039622 oder WO2013066775 beschrieben. In diesem Zusammenhang wurde beobachtet, dass die in der letztgenannten WO2013066775 offenbarte Leckagedetektion mittels eines Mikrophons noch weiter verbessert werden kann, wenn der Verband zuvor mit einer wässrigen Flüssigkeit, welche ein Netzmittel enthält, besprüht wird.

Gemäß einer besonders bevorzugten Ausführungsform der Erfindung wird ein Kit zum Abdichten eines auf der Wunde befindlichen Unterdruck-Wundverbandes bereitgestellt. Das Kit umfasst eine Sprühdose beinhaltend eine filmbildende Lösung zum Abdichten eines auf der Wunde befindlichen Unterdruck-Wundverbandes, wobei die filmbildende Lösung auf das Abdeckmaterial aufgesprüht werden kann, um Undichtigkeiten im Verband zu versiegeln, sowie ein Mittel zur Detektion einer undichten Stelle am Verband.

### Ausführungsbeispiel

In eine an einem Unterdruck-Wundsimulator (in der WO2010/072349 beschrieben) vorhandene künstliche Wunde (Durchmesser 6 cm) wird eine Schaumstoff-Wundauflage geeigneter Größe eingebracht. Bei dem Schaumstoff handelt es sich um das kommerziell erhältliche Produkt VivanoMed Foam® (Paul Hartmann AG, Deutschland). Die künstliche Wunde wird mit einer Abdeckfolie aus Polyurethan abgedeckt, wobei die Abdeckfolie am Rand der künstlichen Wunde, also auf der Metallplatte festgeklebt wird. Bei der Abdeckfolie handelt es sich um das kommerziell erhältliche Produkt HydroFilm^{®} (Paul Hartmann AG, Deutschland). Auf die Abdeckfolie wird gemäß Gebrauchsanweisung des Herstellers ein Unterdruckanschlussmittel aufgebracht. Bei dem Unterdruckanschlussmittel (Port) handelt es sich um das kommerziell erhältliche Produkt VivanoTec Port® (Paul Hartmann AG, Deutschland). Die am Port vorhandene Unterdruckleitung wird mit einem Luftstrommessgerät von Vögtlin Instruments AG (Schweiz) verbunden. Weiterhin wird das Luftstrommessgerät mit einer Unterdruckquelle verbunden. Bei der Unterdruckquelle handelt es sich um die Unterdruck-Therapieeinheit VivanoTec® (Paul Hartmann AG, Deutschland). Es wird ein kontinuierlicher Unterdruck von 125 mm Hg am Gerät eingestellt. Anschließend wird abgewartet, bis die künstliche Wunde evakuiert ist und am Luftstrommessgerät kein Luftstrom mehr gemessen werden kann. Die darauf folgenden Versuche werden nur mit zunächst vollständig abgedichteten Verbänden durchgeführt (in der medizinischen Praxis ist eine derartige vollständige Abdichtung eines Unterdruckverbandes meist nicht erreichbar). In die Abdeckfolie des an die künstliche Wunde angebrachten Verbandes werden danach jeweils ein bis drei Öffnungen eingebracht. Zur Perforation wird ein Stahl-Stift mit einem Durchmesser von 1 mm bzw. 2 mm eingesetzt. Das Luftstrommessgerät zeigt nun einen Luftstrom an, welcher von der Anzahl und dem Durchmesser der Perforationen abhängt. Bei drei Perforationen pro Verband, wobei die Perforationen jeweils einen Durchmesser von 2 mm aufweisen, kann am Luftstrommessgerät beispielsweise ein Luftstrom von 3,5 I/min gemessen werden. Anschließend wird die Unterdruckeinheit abgeschaltet. Es werden 2 Minuten abgewartet, bis im Wundraum der künstlichen Wunde Umgebungsdruck herrscht. Auf die Löcher wird aus einer Sprühdose aus einem Abstand von 5 cm eine filmbildende Lösung aufgesprüht. Die Dauer eines Sprühstoßes beträgt jeweils 3 Sekunden. Nach dem Aufsprühen wird 1 bis 2 Minuten gewartet, bis die Unterdruckquelle wieder aktiviert wird (125 mm Hg). Die Messung des Luftstromes erfolgt dann nach 2 weiteren Minuten und wird nach einer weiteren Stunde wiederholt.

Mit dem vorstehend beschriebenen Versuchsaufbau können die Auswirkungen folgender Einflussfaktoren auf die Abdichtung des Verbandes untersucht werden:
Anzahl und Durchmesser der im Verband vorhandenen Öffnungen
Art der Abdeckfolie
Art und Zusammensetzung der filmbildenden Lösung
Anzahl und Dauer der Sprühstöße
Abstand der Sprühdose zum Verband
Gegebenenfalls durchgeführte Nachbehandlung, beispielsweise Verreiben der aufgesprühten Lösung
Zeitraum nach dem Auftragen der filmbildenden Lösung bis zum erneuten Einschalten der Unterdruckquelle
Höhe des Unterdrucks

Eine zuverlässige Abdichtung von Perforation mit Durchmessern von 1 mm oder 2 mm konnte beispielsweise bei Verwendung des kommerziell erhältlichen Sprühpflasters, "flint MED Sprühverband" (Togal Werk AG, Deutschland) erreicht werden. Hierzu wurde der Sprühverband ein bis dreimal aus einer Entfernung von ca. 5 cm zur Abdeckfolie aufgesprüht. Eine ausreichende und anhaltende Abdichtung von Perforationen mit 1 mm Durchmesser konnte bereits nach einmaligem Besprühen (Dauer des Sprühstoßes 3 Sekunden) erreicht werden. Eine Abdichtung von Perforationen mit 2 mm Durchmesser konnte beispielsweise durch drei in einem Abstand von jeweils 15 Sekunden aufeinanderfolgende Sprühstöße (Dauer des Sprühstoßes jeweils 3 Sekunden) erreicht werden, wobei es sich als vorteilhaft erwies, die filmbildende Lösung nach dem ersten oder zweiten Besprühen an der Auftragsstelle mit einem nicht saugfähigen Tuch zu verreiben und dann erneut zu übersprühen. Der "flint MED Sprühverband" enthält nach Herstellerangaben die polymerisierbare Substanz Poly-(butylmethacrylat, methylacrylat) sowie die Hilfsstoffe Butan, Ethylacetat, Propan und natives Rizinusöl.

## Patentansprüche

1. **Verbandset,** geeignet zur Anwendung bei der Unterdrucktherapie von Wunden umfassend,
- eine **Wundauflage** zum Einbringen in den Wundraum,
- ein luftundurchlässiges **Abdeckmaterial** zum luftdichten Verschließen der Wunde und der Wundumgebung,
- ein **Unterdruckanschlussmittel** zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind,
- eine **Sprühdose** beinhaltend eine filmbildende Lösung zum Abdichten eines auf der Wunde befindlichen Unterdruck-Wundverbandes, wobei die filmbildende Lösung auf das Abdeckmaterial aufgesprüht werden kann, um Undichtigkeiten im Unterdruck-Wundverband zu versiegeln.

2. **Verbandset** nach Anspruch 1, wobei die filmbildende Lösung eine polymerisierbare Substanz umfasst, welche nach dem Aufsprühen der Lösung auf den Verband einen das Abdeckmaterial versiegelnden Polymerfilm bildet.

3. **Verbandset** nach Anspruch 2, wobei die polymerisierbare Substanz Acrylsäure, Methacrylsäure, Vinylchlorid, ein Monosaccharid, ein Disaccharid oder ein Derivat dieser Stoffe umfasst.

4. **Verbandset** nach Anspruch 2 oder 3, wobei der durch die polymerisierbare Substanz gebildete Polymerfilm Nitrocellulose, Polyurethan, Polyurethan-Copolymer, Polyurethan-Polyharnstoff-Copolymer, Polyacrylat, Polymethacrylat oder ein Copolymer auf Acrylat oder Methacrylatbasis umfasst.

5. **Verbandset** nach Anspruch 2, wobei der durch die polymerisierbare Substanz gebildete Polymerfilm ein natürliches Elastomer umfasst.

6. **Verbandset** nach einem der vorangehenden Ansprüche, wobei die filmbildende Lösung einen Weichmacher, ein Netzmittel und/oder einen Emulgator umfasst.

7. **Verbandset** nach einem der vorangehenden Ansprüche, wobei die filmbildende Lösung eine antibakteriell wirkende Substanz umfasst.

8. **Verbandset** nach einem der vorangehenden Ansprüche, wobei das Verbandset weiterhin ein **Mittel zur Detektion** einer undichten Stelle am Unterdruck-Wundverband umfasst.

9. **Verbandset** nach Anspruch 8, wobei es sich bei dem Mittel zur Detektion einer undichten Stelle am Unterdruck-Wundverband um steriles Wasser oder um eine sterile wässrige Lösung handelt.

10. **Verbandset** nach Anspruch 9, wobei die sterile wässrige Lösung einen Fluoreszenz-Farbstoff oder einen mit bloßem Auge erkennbaren Farbstoff enthält.

11. **Kit** zum Abdichten eines auf der Wunde befindlichen Unterdruck-Wundverbandes, umfassend
- eine Sprühdose beinhaltend eine filmbildende Lösung zum Abdichten eines auf der Wunde befindlichen Unterdruck-Wundverbandes, wobei die filmbildende Lösung auf das Abdeckmaterial aufgesprüht werden kann, um Undichtigkeiten im Unterdruck-Wundverband zu versiegeln,
- ein Mittel zur Detektion einer undichten Stelle am Verband, um eine undichte Stelle im Unterdruck-Wundverband sichtbar zu machen.

12. **Kit** nach Anspruch 11, wobei es sich bei dem Mittel zur Detektion einer undichten Stelle am Unterdruck-Wundverband um eine Sprühvorrichtung mit einer Flüssigkeit handelt.

13. **Verfahren** zum Abdichten eines auf der Wunde befindlichen Unterdruck-Wundverbandes, umfassend die Schritte
i) Erkennen einer undichten Stelle am Unterdruck-Wundverband,
ii) Deaktivieren der Unterdruckquelle und Belüften des Verbandes, so dass im Wundraum weitestgehend Umgebungsdruck vorhanden ist,
iii) Aufsprühen einer filmbildenden Lösung auf die undichte Stelle des Unterdruck-Wundverband,
iv) gegebenenfalls Verreiben der filmbildenden Lösung auf dem Unterdruck-Wundverband,
v) Abwarten für mindestens 30 Sekunden,
vi) Fortsetzen der Unterdrucktherapie.

14. **Verfahren** zum Abdichten eines auf der Wunde befindlichen Unterdruck-Wundverbandes nach Anspruch 13, wobei bei Schritt i) ein Mittel zur Detektion einer undichten Stelle im Unterdruck-Wundverband eingesetzt wird.

15. **Verwendung einer Sprühdose beinhaltend eine filmbildenden Lösung** zum Abdichten eines auf der Wunde befindlichen Unterdruck-Wundverbandes.
